# EUROPÄISCHE PATENTSCHRIFT

(11) **EP 1 326 984 B1**
(45) Veröffentlichungstag und Bekanntmachung des Hinweises auf die Patenterteilung: **28.03.2012**
(21) Anmeldenummer: 01987798.4
(22) Anmeldetag: 16.10.2001
(51) Int. Cl.: C12N 15/53

(54) **CYTOCHROM P450 MONOOXYGENASEN AUS THERMOPHILEN BAKTERIEN**
CYTOCHROME P450 MONOOXYGENASES CONSISTING OF THERMOPHILIC BACTERIA
MONO-OXYGENASES DE TYPE CYTOCHROME P450 CONSTITUEES DE BACTERIES THERMOPHILES

(30) Priorität: 16.10.2000 DE 10051175
(43) Veröffentlichungstag der Anmeldung: 16.07.2003
(73) Patentinhaber: BASF SE, 67056 Ludwigshafen (DE)
(72) Erfinder: HAUER, Bernhard, 67136 Fussgönheim (DE); SCHMID, Rolf, 70329 Stuttgart (DE); MERKL, Rainer, 37120 Bovenden (DE); BLASCO, Francesca, 73734 Esslingen (DE)
(86) Internationale Anmeldenummer: PCT/EP2001/011958
(87) Internationale Veröffentlichungsnummer: WO 2002/033057

(56) Entgegenhaltungen:
- WO-A1-96/14419
- WO-A1-96/27678
- PATENT ABSTRACTS OF JAPAN vol. 004, no. 085 (C-015), 18. Juni 1980 (1980-06-18) & JP 55 048391 A (MITSUBISHI CHEM IND LTD), 7. April 1980 (1980-04-07)
- KATO RYUICHI ET AL: "Characterization of a thermostable DNA photolyase from an extremely thermophilic bacterium, Thermus thermophilus HB27." JOURNAL OF BACTERIOLOGY, Bd. 179, Nr. 20, 1997, Seiten 6499-6503, XP002205721 ISSN: 0021-9193 -& DATABASE EMBL [Online] 29. Oktober 1997 (1997-10-29) Database accession no. AB001637 XP002205722
- DATABASE EMBL [Online] 1. Mai 2000 (2000-05-01) SASAKI T ET AL: "Oryza sativa nipponbare (GA3) genomic DNA, chromosome 6, PC clone: P0514G12" Database accession no. Q9SNG3 XP002205723
- DATABASE EMBL [Online] 1. Oktober 2000 (2000-10-01) ZAMORANO J. P. ET AL: "Isolation and characterization of cDNA for mRNAs regulated during cold storage of avocado fruit" Database accession no. Q9M4L1 XP002205724
- DATABASE EMBL [Online] 1. November 1998 (1998-11-01) ALTENBUCHNER J: "Amplifiable elment AUD4 form Streptomyces lividans 66." Database accession no. O85697 XP002205725
- DATABASE EMBL [Online] 30. Mai 2000 (2000-05-30) FLEISCHMANN R. D. ET AL: "Whole genome comparison of Mycobacterium tuberculosis clinical and laboratory strains" Database accession no. O69653 XP002205726
- PARK H-J ET AL: "PURIFICATION AND CHARACTERIZATION OF A NADH OXIDASE FROM THE THERMOPHILE THERMUS-THERMOPHILUS HB8" EUROPEAN JOURNAL OF BIOCHEMISTRY, Bd. 205, Nr. 3, 1992, Seiten 881-885, XP001084250 ISSN: 0014-2956
- MUNRO A W ET AL: "REGIONAL SATURATION MUTAGENESIS AS AN APPROACH TO IDENTIFICATION OFSUBSTRATE SPECIFICITY DETERMINANTS IN CYTOCHROME P450 BM3", BIOCHEMICAL SOCIETY TRANSACTIONS, PORTLAND PRESS LTD, GB, vol. 21, no. 4, 1 January 1993 (1993-01-01), page 409S, XP000971665, ISSN: 0300-5127

## Beschreibung

Die Erfindung betrifft neuartige Cytochrom P450 Monooxygenasen aus thermophilen Bakterien, insbesondere der Gattung Thermus sp., dafür kodierende Nucleotidsequenzen, die rekombinante Herstellung dieser Monooxygenasen und deren Verwendung zur mikrobiologischen Oxidation organischer Verbindungen.

Cytochrom P450 Monooxygenasen besitzen die Fähigkeit technisch interessante Oxygenierungsreaktionen zu katalysieren und werden daher seit einiger Zeit intensiv untersucht. So wurde beispielsweise die Cytochrom P450 Monooxygenase BM-3 aus *Bacillus megaterium* isoliert und charakterisiert und ist mittlerweile auf rekombinantem Weg zugänglich (vgl. z.B. DE-A-199 35 115).

Diese Cytochrom P450-Monooxygenase katalysiert gewöhnlich die subterminale Hydroxylierung langkettiger, gesättigter Säuren und der entsprechenden Amide und Alkohole davon oder die Epoxydation ungesättigter langkettiger Fettsäuren oder gesättigter Fettsäuren mit mittlerer Kettenlänge. Die optimale Kettenlänge gesättigter Fettsäuren beträgt 14 bis 16 Kohlenstoffatome.

Die Struktur der Häm-Domäne von P450 BM-3 wurde durch Röntgenstruktüranalyse bestimmt. Die Substratbindungsstelle liegt in Form einer langen tunnelartigen Öffnung vor, welche von der Moleküloberfläche bis hin zum Häm-Molekül reicht und wird fast ausschließlich von hydrophoben Aminosäureresten begrenzt. Die einzigen geladenen Reste an der Oberfläche der Häm-Domäne sind die Reste Arg47 und Tyr51. Man nimmt an, daß diese an der Bindung der Carboxylatgruppe des Substrates durch Bildung einer Wasserstoffbrückenbindung beteiligt sind. Durch gezielte Einführung von Punktmutationen ist es zwischenzeitlich gelungen, das Substratspektrum dieses Enzyms zu erweitern. So können nunmehr auch kürzer- als auch längerkettige Carbonsäuren, Alkane, Alkene, Cycloalkane, Cycloalkene und verschiedenste Aromaten durch dieses Enzym oxidiert werden (vgl. DE-A-199 35 115, 199 55 605, 100 11 723 und 100 14 085).

Um die industrielle Anwendbarkeit dieser Enzymklasse weiter zu verbessern, wäre es daher wünschenswert neue Cytochrom P450-Monooxygenasen zu finden, welche besser an industrielle Produktionsbedingungen angepasst sind, wie z.B. Enzyme mit erhöhter thermischer Stabilität.

Aufgabe der vorliegenden Erfindung war daher die Bereitstellung von Cytochrom P450-Monooxygenasen, welche besser an industrielle Produktionsbedingungen angepasst sind

Obige Aufgabe wurde gelöst durch Bereitstellung einer Cytochrom P450 Monooxygenase nach Anspruch 1. Sie kann insbe-sondere eine Teilsequenz von Aminosäurerest Pro328 bis Glu345 gemäß SEQ ID NO:2 und vorzugsweise außerdem eine Teilsequenz von Aminosäurerest Val216 bis Ala227 gemäß SEQ ID NO:2 umfassen.

Offenbart sind Cytochrom P450 Monooxygenasen welche eine Aminosäuresequenz auf weisen welche wenigstens eine weitere Teilsequenz umfasst, die ausgewählt ist unter einer Teilsequenzen von wenigstens 10 aufeinanderfolgenden Aminosäure aus den durch die Aminosäurereste Met1 bis Phe327 und Gly346 bis Ala389 gemäß SEQ ID NO:2 vorgegebenen Sequenzbereichen.

Eine besonders bevorzugte Cytochrom P450 Monooxygenase besitzt eine Aminosäuresequenz, welche im wesentlichen SEQ ID NO: 2 entspricht.

Erfindungsgemäße Cytochrom P450 Monooxygenasen sind insbesondere aus thermophilen Bakterien, vorzugsweise der Gattung Thermus sp., wie z.B. der Spezies Thermus thermophilus, Stamm HB27 (hinterlegt bei der DSM unter der Nummer DSM7039) isolierbar. "thermophile" Bakterien erfüllen erfindungsgemäß die Temperaturtoleranzkriterien nach H.G. Schlegel, Allgemeine Mikrobiologie, Thieme Verlag Stuttgart, 5. Auflage , Seite 173, für thermophile und extrem thermophile Organismen (d.h. Wachstumsoptimum bei über 40 °C).

Die erfindungsgemäßen Monooxygenasen sind vorzugsweise durch eine erhöhte Temperaturstabilität gekennzeichnet. Diese drückt sich in einem in Vergleich zum P450 BM-3 aus *Bacillus megaterium* geringeren Aktivitätsverlust bei erhöhter Temperatur (z.B. in einem Bereich von 30 bis 60 °C, pH 7,5, 25mM Tris/HCl) aus.

Gemäß einer bevorzugten Ausführungsform wird erfindungsgemäß eine Cytochrom P450 Monooxygenase aus dem thermophilen Bakterium *T*. *thermophilus* bereitgestellt. Das Protein besitzt ein Molekulargewicht von etwa 44 kDa (bestimmt durch SDS-Gelelektrophorese), ist löslich und zeigt im reduzierten Zustand, oxidierten Zustand und als Carbonyl-Addukt ein Absorbtionsspektrum analog zu dem anderer P450 Enzyme. Aus Sequenzvergleichen dieses erfindungsgemäßen Enzyms aus T. thermophylus und anderen bekannten P450 Enzymen konnten folgende Identitäten bestimmt werden: P450 BM3, 32% Identität; CYP119, 29% Identität; P450eryF, 31% Identität. Das erfindungsgemäße Enzym zeigt eine außerordentliche Thermostabilität, veranschaulicht durch eine Schmelztemperatur von etwa 85°C, welcher Wert um 30°C Ober demjenigen für P450cam liegt.

Offenbart sind weiterhin Oligonukleotide, welche mit einer Nuklein-säuresequenz hybridisieren, die für eine erfindungsgemäße Cytochrom P450 Monooxygenase kodiert.

Insbesondere offenbart sind auch solche Oligonukleotide, welche eine Nukleinsäuresequenz umfassen, die im wesentlichen komplementär ist zu einem wenigstens 30 bis 45 aufeinanderfolgende Nukleotidreste umfassenden Nukleotidsequenzbereich gemäß SEQ ID NO:1.

Offenbart sind Polynukleotide, welche mit einem Oligonukleotid gemäß obiger Definition hybridisieren und für eine Cytochrom P450 Monooxygenase kodieren, insbesondere eine Cytochrom P450 Monooxygenase aus anderen Mikroorganismen, wie z.B. solchen der Gattung *Thermus* sp.. Gegenstand der Erfindung sind insbesondere auch Polynukleotide, die für eine Cytochrom P450 Monooxygenase gemäß obiger Definition kodieren, sowie das dazu komplementäre Polynukleotid_{.}

Bevorzugte Polynukleotide sind solche, die im wesentlichen eine Nukleinsäuresequenz gemäß SEQ ID NO: 1 besitzen, sowie die dazu komplementäre Nukleinsäuresequenz.

Ein weiterer Gegenstand der Erfindung betrifft Expressionskassetten zur rekombinanten Herstellung erfindungsgemäßer Monooxygenasen, umfassend wenigstens eine regulatorische Nukleinsäuresequenz operativ verknüpft mit wenigstens einer der oben angegebenen Polynukleotide.

Weiter Gegenstände der Erfindung betreffen rekombinanter Vektoren, welche wenigstens ein Polynukleotid oder wenigstens eine Expressionskassette gemäß obiger Definition tragen; sowie Mikroorganismen, enthaltend wenigstens einen solchen rekombinanten Vektor; sowie Verfahren zur Herstellung erfindungsgemäßer Cytochrom P450 Monooxygenasen, bei welchen man einen Mikroorganismus, welcher Cytochrom P450 Monooxygenase produziert, kultiviert und die Monooxygenase aus der Kultur isoliert.

Die erfindungsgemäßen Enzyme und davon ableitbaren Mutanten sind als Biokatalysatoren für unterschiedliche biochemische Oxygenierungsreaktionen organischer Verbindungen gemäβ der Definition in den Anspruchen von technischer Bedeutung brauchbar. In analoger Weise sind auch die erfindungsgemäßen rekombinanten Mikroorganismen zur Durchführung solcher Oxygenierungsreaktionen einsetzbar.

Ein weiterer Gegenstand der Erfindung betrifft daher ein Verfahren zur mikrobiologischen Oxidation einer organischen Verbindung gemäβ der Definition in den Anspruchen wobei man diese Verbindung mit wenigstens einer erfindungsgemäßen Cytochrom P450 Monooxygenase umsetzt.

Vorzugsweise wird dieses Verfahren so durch geführt, dass man
a1) einen rekombinanten Mikroorganismus gemäß obiger Definition in einem Kulturmedium, in Gegenwart der exogenen (von außen zugesetzten) oder intermediär gebildeten organischen Verbindung, welche ein Substrat der Monooxygenase ist, vorzugsweise in Gegenwart von Sauerstoff und gegebenenfalls einem Elektronendonor, kultiviert; oder
a2) ein Substrat-haltiges Reaktionsmedium, vorzugsweise in Gegenwart von Sauerstoff und einem Elektronendonor, mit einer erfindungsgemäßen Cytochrom P450 Monooxygenase inkubiert; und
b) das gebildete Oxidationsprodukt oder ein Folgeprodukt davon aus dem Medium isoliert.

Das exogene oder intermediär gebildete Substrat kann dabei ausgewählt sein unter:
a) gegebenenfalls substituierten N-, O- oder S-heterocyclischen ein-, zwei- oder mehrkernigen aromatischen Verbindungen; (nicht Gegenstand der vorliegenden Erfindung;)
b) gegebenenfalls substituierten ein- oder mehrkemigen Aromaten; (nicht Gegenstand der vorliegenden Erfindung;)
c) geradkettigen oder verzweigten Alkanen und Alkenen (nicht Gegenstand der vorliegenden Erfindung;)
d) gegebenenfalls substituierten Cycloalkanen und Cycloalkenen; und
e) aliphatischen, vorzugsweise terminal gesättigten, Carbonsäuren (nicht Gegenstand der vorliegenden Erfindung;)

Nach einer ersten bevorzugten Variante des erfindungsgemäßen Verfahrens wird die Oxidation durch Kultivierung der Mikroorganismen in Gegenwart von Sauerstoff bei einer Kultivierungstemperatur von mindestens etwa 20 °C und einem pH-Wert von etwa 6 bis 9 durchführt.

Nach einer zweiten bevorzugten Variante des erfindungsgemäßen Verfahrens setzt man als exogenes Substrat wenigstens eine Verbindung, ausgewählt unter der oben definierten Gruppe d), einem Medium zu und führt die Oxidation durch enzymatische Umsetzung des substrathaltiges Mediums in Gegenwart von Sauerstoff bei einer Temperatur von mindestens etwa 20°C und einem pH-Wert von etwa 6 bis 9. durch, wobei das substrathaltige Medium außerdem bezogen auf das Substrat einen etwa 10- bis 100-fachen molaren Überschuß an Reduktionsäquivalenten (Elektronendonor) enthält.

Obige Verfahren können bevorzugt in Bioreaktoren durchgeführt werden. Gegenstand der Erfindung sind daher solche Bioreaktoren, umfassend wenigstens eine erfindungsgemäße Monooxygenase oder wenigstens einen rekombinanten Mikroorganismus, gegebenenfalls jeweils in immobilisierter Form.

Schließlich betrifft die Erfindung die Verwendung einer Cytochrom P450 Monooxygenase, eines Vektors oder eines Mikroorganismus gemäß vorliegender Erfindung zur mikrobiologischen Oxidation oben genannter organischer Verbindungsklassen.

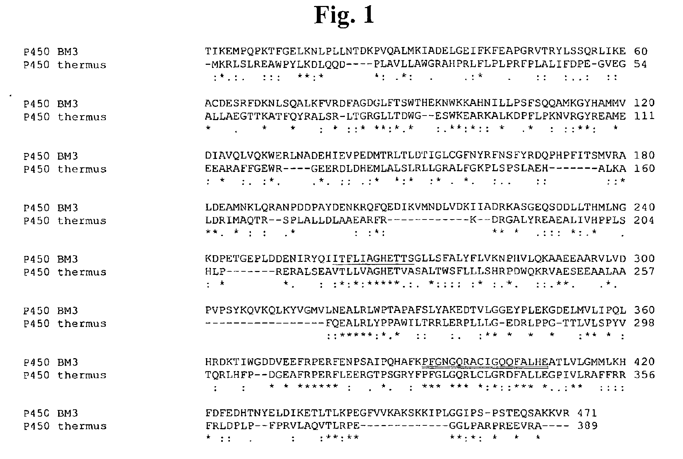

Die Erfindung wird nun unter Bezugnahme auf beiliegenden Figuren näher erläutert. Dabei zeigt
Figur 1 einen Sequenzvergleich von P450 aus *Thermus thermophilus* mit der Häm-Domäne von P450 BM3 aus *Bacillus megaterium.* Doppelt unterstrichen ist dabei die Häm-Bindungsstelle gezeigt (Cys400 in P450 BM3 ist der Cysteinrest, der mit dem Eisenatom der prosthetischen Gruppe koordiniert). Einfach unterstrichen ist die Region die in Kontakt steht mit dem ω-Ende der Fettsäurekette. Die Grad der Übereinstimmung ist durch verschiedenen Symbole gekennzeichnet("*" = identische Reste; ":"rund"." = ähnliche Reste).

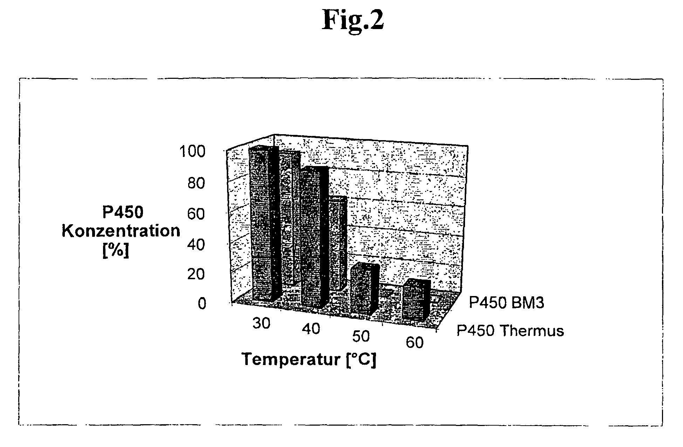

Figur 2 zeigt das Ergebnis eines Vergleichstests zzur Bestimmung der Thermostabilität von P450 BM3 und P450 aus *Thermus* sp.. Die Thermostabilität wurde spektrometrisch im Wellenlängenbereich zwischen 400 und 500nm über den Häm-Gruppen-Gehalt bestimmt.

Offenbart sind ebenfalls "funktionale Äquivalente" der konkret offenbarten neuen P450 Monooxygenasen.

"Funktionale Äquivalente" oder Analoga der konkret offenbarten Monooxygenasen sind davon verschiedene Enzyme, welche weiterhin die gewünschte Substratspezifität im Rahmen der oben bezeichneten Oxidationsreaktion d) besitzen und /oder im Vergleich zu P450 BM3 eine erhöhte Thermostabilität, z.B. bei Temperaturen im Bereich von etwa 30 bis 60 °C und gegebenenfalls höheren Temperaturen nach 30-minütiger Behandlung in 25mM Tris/HCl, besitzen.

Unter "funktionalen Äquivalenten" versteht man erfindungsgemäß insbesondere Mutanten, welche in wenigstens einer der oben genannten Sequenzpositionen eine andere als die konkret genannte Aminosäure aufweisen aber trotzdem eine der oben genannten Oxidationsreaktionen katalysieren. "Funktionale Äquivalente" umfassen 1 bits 30 oder 1 bis 20 oder 1 bis 10, Aminosäure-Additionen, -Substituenten, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen.Funktionale Äquivalenz ist insbesondere auch dann gegeben, wenn die Reaktivitätsmuster zwischen Mutante und unverändertem Enzym qualitativ übereinstimmen, d.h. beispielsweise gleiche Substrate mit unterschiedlicher Geschwindigkeit umgesetzt werden.

Erfindungsgemäß mit umfasste "funktionale Äquivalente" weisen eine von SEQ ID NO:2 in mindestens einer Position abweichende Aminosäuresequenz auf, wobei die Veränderung in der Sequenz die Monooxygenase Aktivität vorzugsweise nur unwesentlich, das heißt um nicht mehr als etwa ± 90%, insbesondere ± 50% oder nicht mehr als ± 30% verändert. Diese Veränderung kann unter Verwendung eines Referenzsubstrates, wie zum Beispiel β-Ionon, unter standardisierten Bedingungen (zum Beispiel 0,1 bis 0,5 M Substrat, pH-Bereich 6 bis 8, insbesondere 7; T = 60 bis 70°C, insbesondere 65°C) bestimmt werden.

"Funktionale Äquivalente" sind Homologe zu den konkret offenbarten Protonen. Diese besitzen wenigstens 60 %, vorzugsweise wenigstens 75% ins besondere wenigsten 85 %, wie z.B. 90%, 95% oder 99%, Homologie zu einer der konkret offenbarten Sequenzen, berechnet nach dem Algorithmus von Pearson und Lipman, Proc. Natl. Acad, Sci. (USA) 85(8), 1988, 2444-2448.

Homologe der erfindungsgemäßen Proteine oder Polypeptide können durch Mutagenese erzeugt werden, z.B. durch Punktmutation oder Verkürzung des Proteins.

Homologe des erfindungsgemäßen Proteine können durch Screening kombinatorischer Banken von Mutanten, wie z.B. Verkür-zungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen codieren. Verfahren zur Synthese degenerierter Oligonukleotide sind dem Fachmann bekannt (Z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

"Funktionale Äquivalente" umfassen natürlich auch P450-Monooxygenasen, welche aus anderen Organismen, z.B. aus anderen als den hierin konkret genannten Bakterien, zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

Oxidierbare Substrate der Gruppe a) sind gegeberienfalls substituierte heterocyclische ein-, zwei- oder mehrkernigen aromatischen Verbindungen; insbesondere oxidierbare oder hydroxylierbare N-, O- oder S-heterocyclische ein-, zwei- oder mehrkernige aromatische Verbindungen. Sie umfassen z.B. zwei oder drei vier- bis siebengliedrige, insbesondere sechs- oder fünfgliedrige, kondensierte Ringe, wobei wenigstens einer, vorzugsweise alle Ringe aromatischen Charakter besitzen und wobei wenigstens einer der aromatischen Ringe ein bis drei, vorzugsweise ein N-, O- oder S-Heteroatom im Ring trägt. In der gesamten Ringstruktur können gegebenenfalls ein oder zwei weitere gleiche oder verschiedene Heteroatome enthalten sein. Die aromatischen Verbindungen können weiterhin 1 bis 5 Substituenten an den Ring-Kohlenstoff oder an den Heteroatomen tragen. Beispiele für geeignete Substituenten sind C₁ bis C₄-Alkyl, wie Methyl, Ethyl, n- oder i-Propyl oder n-, i- oder t- Butyl oder C₂ bis C₄-Alkenyl, wie Ethenyl, 1-Propenyl, 2-Propenyl, 1-Butenyl, 2-Butenyl oder 3-Butenyl, Hydroxyl und Halogen, wie F, Cl, und Br. Die genannten Alkyl- oder Alkonylsubstituenten können gegebenenfalls auch eine Keto-oder Aldehydgruppe aufweisen; Beispiele hierfür sind Propan-2-on-3-yl, Butan-2-on-4-yl, 3-Buten-2-on-4-yl. Nichtlimitierende Beispiele für geeignete heterocyclische Substrate sind insbesondere zweikernige Heterocyclen, wie Indol, N-Methylindol und die mit ein bis drei Substituenten an Kohlenstoffatomen substituierten Analoga davon, wie z.B. 5-Chlor- oder 5-Brom-indol; sowie Chinolin und Chinolinderivate, wie z.B. 8-Methylchinolin, 6-Methylchinotin und Chinaldin; und Benzothiophen und die mit ein bis drei Substituenten an Kohlenstoffatomen substituierten Analoga davon. Außerdem seien genannt dreikemige Heteroaromaten, wie Acridin, und die mit ein bis drei Substituenten an Kohlenstoffatomen substituierten Analoga davon.

Oxidierbare Substrate der Gruppe b) sind gegebenenfalls substituierte ein- oder mehrkernige, insbesondere ein- oder zweikernige Aromaten, wie Benzol und Naphthalin. Die aromatischen Verbindungen können gegebenenfalls ein oder mehrfach substituiert sein und z.B. 1 bis 5 Substituenten an den RingKohlenstoffatomen tragen. Beispiele für geeignete Substituenten sind C₁ bis C₄-Alkyl, wie Methyl, Ethyl, n- oder i-Propyl oder n-, i- oder t- Butyl, oder C₂ bis C₄-Alkenyl, wie Ethenyl, 1-Propenyl, 2-Propenyl, 1-Butenyl, 2-Butenyl oder 3-Butenyl, Hydroxyl und Halogen, wie F, Cl, und Br. Die genannten Alkyl- oder Alkenylsubstituenten können gegebenenfalls auch eine Keto- oder Aldehydgruppe aufweisen; Beispiele hierfür sind Propan-2-on-3-yl, Butan-2-on-4-yl, 3-Buten-2-on-4-yl. Der Aromat kann gegebenenfalls mit einem vier- bis siebengliedrigen, nichtaromatischen Ring kondensiert sein. Der nichtaromatische Ring kann gegebenenfalls eine oder zwei C-C-Doppelbindungen aufweisen, ein- oder mehrfach mit oben genannten Substituenten substituiert sein und gegebenenfalls ein oder zwei Ringheteroatome tragen. Beispiele für besonders brauchbare Aromaten sind einkernige Aromaten, wie Cumol, sowie zweikernige Substrate, wie Inden und Naphthalin, sowie die mit ein bis drei Substituenten an Kohlenstoffatomen substituierten Analoga davon.

Oxidierbare Substrate der Gruppe c) sind geradkettige oder ver zweigte Alkane oder Alkene mit 4 bis 15, vorzugsweise 6 bis 12 Kohlenstoffatomen. Als Beispiele können genannt werden n-Pentan, n-Hexan, n-Heptan-, n-Oktan, n-Nonan, n-Decan, n-Undecan und n-Dodecan, sowie die ein- oder mehrfach verzweigten Analoga dieser Verbindungen, wie z.B. analoge Verbindungen mit 1 bis 3 Methyl-Seitengruppen; oder die ein- oder mehrfach, beispielsweise einfach ungesättigten Analoga der oben genannten Alkane.

Erfindungsgemäß oxidierbare Substrate der Gruppe d) sind gegebenenfalls substituierte Cycloalkane und Cycloalkene. Beispiele hierfür sind Cyclopentan, Cyclopenten, Cyclohexan, Cyclohexen, Cycloheptan und Cyclohepten. Die Ringstruktur kann dabei ein- oder mehrfach substituiert sein und z.B. 1 bis 5 Substituenten gemäß obiger Definition für Verbindungen der Gruppen a) und b) tragen. Nichtlimitierendes Beispiel hierfür sind Ionone, wie α - β - und δ- Ionon, sowie die entsprechenden Methylionone und Isomethylionone.

Oxidierbare, Substrate der Gruppe e) sind geradkettige oder verzweigte, gesättigte oder ein- oder mehrfach ungesättigte C₈-C₃₀-Carbonsäuren, insbesondere Monocarbonsäuren, oder Carbonsäurederivate davon, wie, Ester und Amide. Als Beispiele sind terminal oder subterminal ω-1-,ω-2- oder ω-3-Position) hydroxylierbare gesättigte Monocarbonsäuren zu nennen.

Gegenstand der Erfindung sind auch Nukleinsäuresequenzen (einzel- und doppelsträngige DNA- und RNA-Sequenzen), kodierend für eine der obigen Monooxygenasen und deren funktionale Äquivalente. Weitere erfindungsgemäße Nukleinsäuresequenzen sind abgeleitet von SEQ ID NO:1 und unterscheiden sich davon durch Addition, Substitution, Insertion oder Deletion einzelner oder mehrerer Nukleotide, kodieren aber weiterhin für eine Monooxygenase mit der gewünschten Eigenschaftsprofil.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten, davon. Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Weiterhin offenbart sind auch Nukleinsäuresequenzen, welchen mit oben genannten kodierenden Sequenzen hybridisieren oder dazu komplementär sind. Diese Polynukleotide lassen sich bei Durchmusterung von genomischen oder cDNA-Bibliotheken auffinden und gegebenenfalls daraus mit geeigneten Primern mittels PCR vermehren und anschließend beispielsweise mit geeigneten Sonden isolieren. Eine weitere Möglichkeit bietet die Transformation geeigneter Mikroorganismen mit erfindungsgemäßen Polynukleotiden oder Vektoren, die Vermehrung der Mikroorganismen und damit der Polynukleotide und deren anschließende Isolierung. Darüber hinaus können erfindungsgemäße Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bildungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicher weise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1 x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden.

Gegenstand der Erfindung sind außerdem Expressionskonstrukte, enthaltend unter der genetischen Kontrolle regulativer Nukleinsäuresequenzen eine für eine erfindungsgemäße Mutante kodierende Nukleinsäuresequenz; sowie Vektoren, umfassend wenigstens eines dieser Expressionskonstrukte. Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind . Targeting-Sequenzen sowie Translatlonsverstärker, Enhancer, Polyadenylierungssignale und dergleichen. Weitere regulative Elemente umfassen selektierbare Marker, Amplifikationssignale, Replikationsursprünge und dergleichen.

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, lpp-, lac-, lpplac-, laclq-, T7- T5-, T3-, gal-, trc-, ara-, SP6-, I-PR- oder im I-PL-Promotor, die vorteilhafterweise in gram-negativen Bakterien Anwendung finden; sowie die grampositiven Promotoren amy und SPO2, die Hefepromotoren ADC1, MFa , AC, P-60, CYC1, GAPDH oder die Pflanzenpromotoren CaMV/35S, SSU, OCS, lib4, usp, STLS9, B33, not oder der Ubiquitin- oder Phaseolin-Promotor. Besonders bevorzugt ist die Verwendung induzierbarer Promotoren, wie z:B. licht- und insbesondere temperaturinduztierbarer Promotoren, wie der PᵣP₁Promotor.

Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und die Proteinexpression ermöglichen. Dies kann beispielsweise je nach Wirtsorganismus bedeuten, dass das Gen erst nach Induktion exprimiert oder überexprimiert wird, oder dass es sofort exprimlert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten Monooxygenase-Nukleotidseqüenz sowie einen Terminator- oder Polyadenylierungssignal. Dazu verwendet man gängige Rekombinations- und Klanierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspeziflschen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Plasmide, Cosmide, und lineare oder zirkuläre DNA zu verstehen. Diese Vektoren können autonom im Wirtsorganismus repliziert oder chromosomal repliziert werden.

Mit Hilfe der erfindungsgemäßen Vektoren sind rekombinante Mikroorganismen herstellbar, welche beispielsweise mit wenigstens einem erfindungsgemäßen Vektor transformiert sind und zur Produktion der Mutanten eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z. B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9 oder CHO-Zellen.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Die Kombination aus den Wirtsorganismen und den zu den Organismen passenden Vektoren, wie Plasmide, Viren oder Phagen, wie beispielsweise Plasmide mit dem RNA-Polymerase/Promoter-System, die Phagen λ oder µ oder andere temperente Phagen oder Transposons und/oder weiteren vorteilhaften regulatorischen Sequenzen bildet ein Expressionssystem. Beispielsweise ist unter dem Begriff "Expressionssystem" die Kombination aus Säugetierzellen, wie CHO-Zellen, und Vektoren, wie pcDNA3neo-Vektor, die für Säugetlerzellen geeignet sind, zu verstehen.

Gewünschtenfalls kann das Genprodukt auch in transgenen Organismen wie transgenen Tieren, wie insbesondere Mäusen oder Schafen oder transgenen Pflanzen zur Expression gebracht werden.

Gegenstand der Erfindung sind weiterhin Verfahren zur rekombinanten Herstellung einer erfindungsgemäßen Monooxygenase, wobei man einen Monooxygenaseproduzierenden Mikroorganismus kultiviert, gegebenenfalls die Expression der Monooxygenase induziert und die Monooxygenase aus der Kultur isoliert. Die Monooxygenase kann so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Monooxygenase nicht in das Kulturmedium sezerniert wird, aufgeschlossen und das Enzym nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Monooxygenase kann mit bekannten, chromatographischeri Verfahren erzielt werden, wie Molekularsieb-Chromatographie (Gelfltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Besonders vorteilhaft ist es, zur Isolierung des rekombinanten Proteins Vektorsysteme oder Oligonukleotide zu verwenden, die die cDNA um bestimmte Nucleotidsequenzen verlängern und damit für veränderte Polypeptide oder Fusionsproteine kodieren, die einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Die Erfindung betrifft außerdem ein Verfahren zur mikrobiologischen Oxidation organischer Verbindungen obigen Typs.

Wird die Umsetzung mit einem rekombinanten Mikroorganismus durchgeführt, so erfolgt vorzugsweise zunächst die Kultivierung der Mikroorganismen in Gegenwart von Sauerstoff und in einem Komplexmedium, wie z.B. TB- oder LB- Medium bei einer Kultivierungstemperatur von etwa 20 °C oder mehr, und einem pH-Wert von etwa 6 bis 9, bis eine ausreichende Zelldichte erreicht ist. Um die Oxidationsreaktion besser steuern zu können, bevorzugt man die Verwendung eines induzierbaren Promotors. Die Kultivierung wird nach Induktion der Monooxygenaseproduktion in Gegenwart von Sauerstoff 12 Stunden bis 3 Tage fortgesetzt.

Wird die erfindungsgemäße Umsetzung dagegen mit gereinigtem oder angereichertem Enzym durchgeführt so löst man das erfindungsgemäße Enzym in einem exogenes Substrat enthaltenden Medium (etwa 0,01 bis 10 mM, oder 0,05 bis 5 mM), und führt die Umsetzung, vorzugsweise in Gegenwart von Sauerstoff, bei einer Temperatur von etwa 10 °C oder mehr, und einem pH-Wert von etwa 6 bis 9 (wie z.B. eingestellt mit 100 bis 200 mM Phosphat- oder Tris-Puffer), sowie in Gegenwart eines Reduktionsmittels durch, wobei das Substrat-haltige Medium außer dem bezogen auf das zu oxidierende Substrat einen etwa 10-bis 100-fachen molaren Überschuß an Reduktionsäquivalenten enthält. Bevorzugtes Reduktionsmittel ist NADPH.

Beim erfindungsgemäßen Substratoxidationsprozess wird im Reaktionsmedium enthaltener oder zugesetzter Sauerstoff reduktiv enzymatisch gespalten. Die erforderlichen Reduktionsäquivalente werden von dem zugesetzten Reduktionsmittel (Elektronendonor) zur Verfügung gestellt.

Das gebildete Oxidationsprodukt kann dann in herkömmlicher Weise, wie z.B. durch Extraktion oder Chromatographie, vom Medium abgetrennt und gereinigt werden.

Folgende nichtlimitierende Beispiele beschreiben spezielle Ausführungsformen der Erfindung.

Allgemeine experimentelle Angaben:

### a) Allgemeine Klonierungsverfahren

Die im Rahmen der vorliegenden Erfindung durchgeführten Klonierungsschritte wie z.B. Restriktionsspaltungen, Agarose Gelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vennehrung von Phagen und Sequenzanalyse rekombinanter DNA wurden wie bei Sambrook et al. (1989) a.a.O. beschrieben durchgeführt.

### b) Polymerasekettenreaktion (PCR)

PCR wurde nach Standardprotokoll mit folgendem Standardansatz durchgeführt:

8 µl dNTP-Mix (200µM), 10 µl Taq-Polymerase-Puffer (10 x) ohne MgCl₂, 8µl MgCl₂ (25mM), je 1 µl Primer (0,1 µM), 1µl zu amptifizierende DNA, 2,5 U Taq-Potymerase (MBI Fermentas, Vilnius, Litauen), ad 100 µl demineralisiertes Wasser.

### c) Kultivierung von E.coli

Die Kultivierung von rekombinanten E. coli-Stämme DH5V wurde in LB-Amp. Medium (Trypton 10,0g, NaCl 5,0 g, Hefeextrakt 5,0 g, Ampicillin 100 g/ml H₂0 ad 1000 ml) bei 37°C kultiviert. Dazu wurde jeweils eine Kolonie mittels Impföse von einer Agarplatte in 5 ml LB-Amp überführt. Nach ca. 18 h Stunden Kultivierung bei einer Schüttelfrequenz von 220 Upm wurden 400 ml Medium in einem 2-I-Kolben mit 4 ml Kultur inokuliert. Die Induktion der P450-Expression in E. coli erfolgte nach Erreichen eines OD578-Wertes zwischen 0,8 und 1,0 durch eine drei- bis vierstündige Hitzeschockinduktion bei 42 °C.

### d) Zellaufschluß

Zellpellets mit einer Biofeuchtmasse von bis zu 15 g E. coli DH5∀ wurden auf Eis aufgetaut und in 25 ml Kallumphosphat-Puffer (50 mM, pH 7,5, 1 mM EDTA) oder Tris/HCl Puffer (50 mM, pH 7,5, 1 mM EDTA) suspendiert Mittels dreiminütiger Ultraschallbehandlung (Branson Sonifier W250, (Dietzenbach, Deutschland), Leistungsabgabe 80 W, Arbeitsintervall 20 %) wurde die auf Eis gekühlte E. coli-Zellsuspension aufgeschlossen. Vor der Proteinreinigung wurde die Zellsuspension für 20 min bei 32 500 g zentrifugiert und durch einen 0,22 mm Sterivex-GP-Filter . (Millipore) filtriert, wobei man einen Rohextrakt erhält.

### Beispiel 1:

### Klonierung und Expression von P450 aus Thermus thermophilus HB27 und den Histag.Derivaten davon

### 1. Kionierung von P450 aus Thermus thermophilus HB27

Die kodierende P450-Sequenz (blunt ended) wurde in die Hindi-Schnittsteiie des Plasmids pTZ19R (MBI Fermentas) einkloniert. Aus dem so erhaltenen Plasmid TTHB66 wurde die kodierende P450-Sequenz mit Hilfe der PCR amplifiziert. Dazu wurden folgende Primer verwendet:
a) 30-mer sense-Oligonucleotid, enthaltend die Ndel-Schnittstelle (kursiv gedruckt) als Teil des P450-ATG-Startcodons:
   5'-CGAAGCTCATATGAAGCGCCTTTCCCTGAG (SEQ ID NO:7).
b) 30-mer antisense-Oligonucleotid, enthaltend die EcoRI-Schnittstelle (kursiv gedruckt) als Teil des TGA-Stopcodons: 5'-GCGAATTCACGCCCGCACCTCCTCCCTAGG (SEQ ID NO:8).

Das resultierende Fragment wurde in die Ndel-Schnittstellen des Vektors pCYTEXP1 (Plasmid mit dem temperaturinduzierbaren P_{R}P_{L}-Promotorsystem des Bakteriophagen 8 (Belev T.N., et al., Plasmid (1991) 26:147)) kloniert und in E. coli DH-5α (Clontech, Heidelberg) transformiert.

E. coli DH-5α, enthaltend das interessierende Plasmid wurde in LB-Medium in Gegenwart von Ampicillin inokuliert und die Kultur wurde über Nacht bei 37 °C inkubiert. Ein Teil der Probe wurde in frisches LB-Medium (in Gegenwart von Ampicillin) inokuliert und die resultierende Kultur wurde bei 37 °C bis zu OD = 0,9 kultiviert. Die Induktion erfolgte durch Erhöhung der Temperatur auf 42 °C über einen Zeitraum von 24 Stunden. Die Veränderung des P450-Gehaltes während der Expression wurde anhand von Messungen des CO-Differenzspektrums bestimmt.

| Expressionszeit [h] | ΔA₄₅₀₋₄₉₀ | P450 Konzentration [µM] |
|---|---|---|
| 4 | 0,092 | 0,056 |
| 8 | 0,176 | 0,106 |
| 24 | 0,106 | 0,064 |

### 2. Klonierung von P450 aus Thermus thermophilus HB27 mit N-terminalem Histag

Die kodierende P450-Sequenz wurde durch PCR aus dem Plasmid TTHB66 unter Verwendung folgender Primer amplifiziert:
(a) 50-mer sense-Oligonucleotid, enthaltend die Ndel-Schnittstelle (kursiv gedruckt) als Teil des P450 ATG-Startcodons und die tag-codierenden Codons (unterstrichen):
   5'-CGAAGCT***CATATG***CATCACCATCATCATCACAAGCGCCTTTC (SEQ ID NO:9);
(b) 30-mer antisense-Oligonucleotid, enthaltend die EcoRI-Schnittstelle (kursiv gedruckt) als Teil des TGA-Stop-Codons :
   5'-GC**G*AATTC***ACGCCCGCACCTCCTCCCTAGG (SEQ ID NO:8).

Das resultierende Fragment wurde in die Ndel- und EcoRI-Schnittstellen des Vektors p-CYTEXP1 kloniert und in E. coli DH-5α exprimiert.

E. coli DH-5α, enthaltend das interessierende Plasmid, wurde in LB-Medium in Gegenwart von Ampicillin inokuliert und die Kultur wurde über Nacht bei 37 °C inkubiert. Ein Teil der Probe wurde in frisches LB-Medium (in Gegenwart von Ampicillin) inokuliert und die resultierende Kultur wurde bei 37 °C bis zu OD = 0,9 kultiviert. Die Induktion erfolgte durch Erhöhung der Temperatur auf 42 °C über einen Zeitraum von 24 Stunden. Die Veränderung des P450-Gehaltes während der Expression wurde anhand von Messungen des CO-Differenzspektrums bestimmt.

| Expressionszeit [h] | ΔA₄₅₀₋₄₉₀ | P450 Konzentration [µM] |
|---|---|---|
| 4 | ND | ND |
| 8 | 0,097 | 0,073 |
| 24 | 0,111 | 0,073 |

### 3. Clonierung von P450 aus Thermus thermophilus HB27 mit C-terminalem Histag

Die kodierende P450-Sequenz wurde durch PCR aus dem Plasmid TTHB66 unter Verwendung der folgenden Primer amplifiziert:
(a) 30-mer sense-Oligonucleotid, enthaltend die Ndel-Schnittstelle (kursiv gedruckt) als Teil des P450 ATG-Start-Codons:
   5'-CGAAGCT***CATATG***AAGCGCCTTTCCCTGAG (SEQ ID NO:7)
(b) 47-mer antisense-Oligonucleotid, enthaltend die EcoRI-Schnittstelle (kursiv gedruckt) als Teil des TGA-Stop-Codons sowie die unterstrichene tagcodierende Teilsequenz:
   5'-CG**G*AATTC***AGTGATGATGATGGTGATGCGCCCGCACCTCCTC (SEQ ID NO:10).

Das resultierende Fragment wurde in die Ndel- und EcoRI-Schniftstellen des Vektors p-CYTEXP1 cloniert und in E. coli DH-5α exprimiert.

E. coli DH-5α, enthaltend das interessierende Plasmid wurde in LB-Medium in Gegenwart von Ampicillin inokuliert und die Kultur wurde über Nacht bei 37 °C inkubiert. Ein Teil der Probe wurde In frisches LB-Medium (in Gegenwart von Ampicillin) inokuliert und die resultierende Kultur wurde bei 37 °C bis zu OD = 0,9 kultiviert. Die Induktion erfolgte durch Erhöhung der Temperatur auf 42°C über einen Zeitraum von 24 Stunden. Die Veränderung des P450-Gehaltes während der Expression wurde anhand von Messungen des CO-Differenzspektrums bestimmt.

| Expressionszeit [h] | ΔA₄₅₀₋₄₉₀ | P450 Konzentration [µM] |
|---|---|---|
| 4 | ND | ND |
| 8 | 0,1 | 0,075 |
| 24 | ND | ND |

### Beispiel 2:

### Bestimmung der Thermostabilität von P450 aus Thermus thermoahllus im Vergleich zu P450 BM3

Die beiden Enzyme wurden jeweils 30 Minuten in Tris/HCI-Puffer pH 7,5, 25mM bei verschiedenen Temperaturen inkubiert. Die Ansätze wurden anschließend abgekühlt und die P450 Konzentration wurde spektrometrisch bestimmt. Die Ergebnisse sind in folgender Tabelle zusammengefaßt und in Figur 2 graphisch dargestellt.

| Temperatur [°C] | | 30 | 40 | 50 | 60 |
|---|---|---|---|---|---|
| P450 Konzentration [%] | P450 thermus | 100 | 89 | 29 | 22 |
| | P450 BM3 | 92 | 63 | 0 | 0 |

Wie man den Versuchsergebnissen entnimmt, besitzt das erfindungsgemäße Enzym nach 30-minütiger Inkubation bei allen Temperaturen eine signifikant höherer Temperaturstabilität.

### Beispiel 3:

### Biotransformationsexaerimente

Der endogene Redoxpartner für die erfindungsgemäße Cytochrom P450 aus T. *thermophilus* konnte bisher noch nicht zweifelsfrei identifiziert werden. Es konnte jedoch beispielsweise Enzymaktivität bei der Hydroxylierung von β- und/oder α-lonon beobachtet werden. Mit β-Ionon als Substrat konnte die Umwandlung zu einem Hauptprodukt beobachtet werden, wohingegen α-Ionon zu einem Produktgemisch umgesetzt wurde. Durch Vergleich mit synthetischen Standards konnte festgestellt werden, daß das Hauptprodukt der β-Ionon-Umwandlung 4-Hydroxy-β-Ionon ist.

Vorkulturen von *T. thermophilus* [5 ml Medium Tt (2 g Hefeextrakt, 1 g Trypton, 1 g NaCl in 500 ml entionisiertem Wasser)] wurden aus Agarplatten-Kulturen inokuliert und 24 Stunden bei 65°C unter Schütteln (150 Upm) inkubiert. Anschließend wurde 100 ml Medium Tt mit der Vorkultur inokuliert und bei 65°C unter Schütteln inkubiert. β-Ionon (107 µl/ml Kultur) wurde jeder Kultur nach 24 Stunden zugesetzt. Die Kultivierung wurde 78 Stunden fortgesetzt. Die Zellen wurden anschließend abzentrifugiert und der Überstand mit Diethyläther extrahiert. Der Extrakt wurde durch GC und TLC analysiert. Kontrollkulturen ohne Substrat wurden unter den gleichen Bedingungen hergestellt und analysiert.

## Patentansprüche

1. Cytochrom P450 Monooxygenase, **dadurch gekennzeichnet, dass** sie eine Aminosäuresequenz gemäß SEQ ID NO:2 aufweist, sowie funktionale Äquivalente davon,
welche sich von SEQ ID NO:2 durch 1 bis 30 Aminosäure-Additionen, -Substitutionen, -Deletionen und/oder -Inversionen unterscheiden, und wobei die Veränderung in der Sequenz die Monooxygenase-Aktivität um nicht mehr als ± 90%, bestimmt unter Verwendung des Referenzsubstrates β-Ionon verändert, wobei die Aktivität unter standardisierten Bedingungen, nämlich 0,1 bis 0,5 M Substrat, pH = 6 bis 8 und T = 60 -70°C, bestimmt wird.

2. Cytochrom P450 Monooxygenase nach Anspruch 1 aus Bakterien der Gattung *Thermus sp..*

3. Cytochrom P450 Monooxygenase nach Anspruch 2, aus einem Bakterium der Spezies *Thermus thermophilus.*

4. Polynukleotid, das für eine thermostabile Cytochrom P450 Monooxygenase gemäß einem der Ansprüche 1 bis 3 kodiert, sowie das dazu komplementäre Polynukleotid.

5. Polynukleotid nach Anspruch 4 mit einer Nukleinsäuresequenz gemäß SEQ ID NO: 1, sowie die dazu komplementäre Nukleinsäuresequenz.

6. Expressionskassette, umfassend wenigstens eine regulatorische Nukleinsäuresequenz operativ verknüpft mit einem Polynukleotid gemäß einem der Ansprüche 4 oder 5.

7. Rekombinanter Vektor, der ein Polynukleotid gemäß einem der Ansprüchen 4 oder 5 oder eine Expressionskassette gemäß Anspruch 6 trägt.

8. Rekombinanter Mikroorganismus, transformiert oder transfiziert mit wenigstens einen rekombinanten Vektor gemäß Anspruch 7.

9. Verfahren zur Herstellung einer thermostabilen Cytochrom P450 Monooxygenase gemäß einem der Ansprüche 1 bis 3, wobei man einen rekombinanten Mikroorganismus nach Anspruch 8, welcher die thermostabile Cytochrom P450 Monooxygenase produziert, kultiviert und die Monooxygenase aus der Kultur isoliert.

10. Verfahren zur mikrobiologischen Oxidation einer organischen Verbindung ausgewählt unter gegebenenfalls substituierten Cycloalkanen und Cycloalkenen, wobei man diese Verbindung mit wenigstens, einer Cytochrom P450 Monooxygenase nach einem der Ansprüche 1 bis 3 umsetzt.

11. Verfahren nach Anspruch 10, **dadurch gekennzeichnet, dass** man
a1) einen rekombinanten Mikroorganismus nach Anspruch 8 in einem Kulturmedium, in Gegenwart der exogenen oder intermediär gebildeten, unter gegebenenfalls substituierten Cycloalkanen und Cycloalkenen ausgewählten organischen Verbindung, welche ein Substrat der Monooxygenasen ist, kultiviert; oder
a2) ein Substrat-haltiges Reaktionsmedium mit einer Cytochrom P450 Monooxygenase nach einem der Ansprüche 1 bis 3 inkubiert; und
b) das gebildete Oxidationsprodukt oder ein Folgeprodukt davon aus dem Medium isoliert.

12. Verfahren nach Anspruch 11, **dadurch gekennzeichnet, dass** man die Oxidation durch Kultivierung der Mikroorganismen in Gegenwart von Sauerstoff bei einer Kultivierungstemperatur von mindestens etwa 20°C und einem pH-Wert von etwa 6 bis 9 durchführt.

13. Verfahren nach Anspruch 11, **dadurch gekennzeichnet, dass** man als exogenes Substrat wenigstens eine Verbindung, ausgewählt unter gegebenenfalls substituierten Cycloalkanen und Cycloalkenen, einem Medium zusetzt und die Oxidation durch enzymatische Umsetzung des substrathaltiges Mediums in Gegenwart von Sauerstoff bei einer Temperatur von mindestens etwa 20°C und einem pH-Wert von etwa 6 bis 9 durchführt, wobei das substrathaltige Medium außerdem bezogen auf das Substrat einen etwa 10-bis 100-fachen molaren Überschuß an Reduktionsäquivalenten enthält.

14. Bioreaktor, umfassend ein Enzym nach einem der Ansprüche 1 bis 3 oder einen rekombinanten Mikroorganismus nach Anspruch 8 in immobilisierter Form.

15. Verwendung einer Cytochrom P450 Monoxygenase nach einem der Ansprüche 1 bis 3, eines Vektors nach Anspruch 7, oder eines Mikroorganismus nach Anspruch 8 zur mikrobiologischen Oxidation von gegebenenfalls substituierten' Cycloalkanen und Cycloalkenen.

## Claims

1. A cytochrome P450 monooxygenase which exhibits an amino acid sequence as shown in SEQ ID No.: 2, and functional equivalents thereof which differ from SEQ ID No.: 2 in 1 to 30 amino acid additions, substitutions, deletions and/or inversions, and where the modification in the sequence alters the monooxygenase activity by no more than ±90%, determined using the reference substrate β-ionone, where the activity is determined under standardized conditions, which are 0.1 to 0.5 M substrate, pH = 6 to 8 and T = 60-70°C.

2. The cytochrome P450 monooxygenase according to claim 1 from bacteria of the genus *Thermus sp..*

3. The cytochrome P450 monooxygenase according to claim 2 from a bacterium of the species *Thermus thermophilus.*

4. A polynucleotide which codes for a thermostable cytochrome P450 monooxygenase according to any of claims 1 to 3, and the polynucleotide which is complementary thereto.

5. The polynucleotide according to claim 4 with a nucleic acid sequence as shown in SEQ ID No.: 1, and the nucleic acid sequence which is complementary thereto.

6. An expression cassette, comprising at least one regulatory nucleic acid sequence in operative linkage with a polynucleotide according to either of claims 4 and 5.

7. A recombinant vector which harbors a polynucleotide according to either of claims 4 and 5 or an expression cassette according to claim 6.

8. A recombinant microorganism, transformed or transfected with at least one recombinant vector according to claim 7.

9. A process for the production of a thermostable cytochrome P450 monooxygenase according to any of claims 1 to 3, wherein a recombinant microorganism according to claim 8 which produces the thermostable cytochrome P450 monooxygenase is cultured and the monooxygenase is isolated from the culture.

10. A process for the microbiological oxidation of an organic compound selected among optionally substituted cycloalkanes and cycloalkenes, wherein this compound is converted with at least one cytochrome P450 monooxygenase according to any of claims 1 to 3.

11. The process according to claim 10, wherein
a1) a recombinant microorganism according to claim 8 is cultured in a culture medium in the presence of the exogenously formed organic compound, or the compound formed as intermediate, which compound is selected among optionally substituted cycloalkanes and cycloalkenes and is a substrate of the monooxygenase enzyme; or
a2) a substrate-comprising reaction medium is incubated with a cytochrome P450 monooxygenase according to any of claims 1 to 3; and
b) the oxidation product formed or a subsequent product thereof is isolated from the medium.

12. The process according to claim 11, wherein the oxidation is carried out by culturing the microorganisms in the presence of oxygen at a culturing temperature of at least approximately 20°C and a pH of approximately 6 to 9.

13. The process according to claim 11, wherein at least one compound selected among optionally substituted cycloalkanes and cycloalkenes is added to a medium as exogenous substrate and the oxidation is carried out by enzymatic conversion of the substrate-comprising medium in the presence of oxygen at a temperature of at least approximately 20°C and a pH of approximately 6 to 9, wherein the substrate-comprising medium additionally comprises an approximately 10- to 100-fold molar excess of reduction equivalents based on the substrate.

14. A bioreactor, comprising an enzyme according to any of claims 1 to 3 or a recombinant microorganism according to claim 8 in immobilized form.

15. The use of a cytochrome P450 Monooxygenase according to any of claims 1 to 3, of a vector according to claim 7 or of a microorganism according to claim 8 for the microbiological oxidation of optionally substituted cycloalkanes and cycloalkenes.

## Revendications

1. Mono-oxygénase à cytochrome P450, **caractérisée en ce qu'**elle présente une séquence d'acides aminés selon SEQ ID N° 2, ainsi que ses équivalents fonctionnels, qui se distinguent de SEQ ID N° 2 par 1 à 30 additions, substitutions, délétions et/ou inversions d'acides aminés, la modification de la séquence ne modifiant pas l'activité de mono-oxygénase de plus de ± 90 %, la détermination s'effectuant par utilisation du substrat de référence β-ionone, l'activité étant déterminée dans des conditions normalisées, à savoir un substrat 0,1 à 0,5 M, pH = 6 à 8, et T = 60 à 70°C.

2. Mono-oxygénase à cytochrome P450 selon la revendication 1, provenant de bactéries du genre *Thermus* sp.

3. Mono-oxygénase à cytochrome P450 selon la revendication 2, provenant d'une bactérie de l'espèce *Thermus thermophilus.*

4. Polynucléotide qui code pour une mono-oxygénase à cytochrome P450 thermostable selon l'une des revendications 1 à 3, ainsi que le polynucleotide qui lui est complémentaire.

5. Polynucléotide selon la revendication 4, ayant une séquence d'acide nucléique selon SEQ ID N° 1, ainsi que la séquence d'acide nucléique qui lui est complémentaire.

6. Cassette d'expression comprenant au moins une séquence d'acide nucléique régulatrice liée d'une manière opérationnelle à un polynucléotide selon l'une des revendications 4 ou 5.

7. Vecteur recombinant, qui porte un polynucléotide selon l'une des revendications 4 ou 5 ou une cassette d'expression selon la revendication 6.

8. Micro-organisme recombinant, transformé ou transfecté avec au moins un vecteur recombinant selon la revendication 7.

9. Procédé de préparation d'une mono-oxygénase à cytochrome P450 thermostable selon l'une des revendications 1 à 3, dans lequel on cultive un micro-organisme recombinant selon la revendication 8, qui produit la mono-oxygénase à cytochrome P450 thermostable, et on isole la mono-oxygénase de la culture.

10. Procédé d'oxydation microbiologique d'un composé organique choisi parmi les cycloalcanes et les cycloalcènes éventuellement substitués, dans lequel on fait réagir ce composé avec au moins une mono-oxygénase à cytochrome P450 selon l'une des revendications 1 à 3.

11. Procédé selon la revendication 10, **caractérisé en ce que**
a1) on cultive un micro-organisme recombinant selon la revendication 8 dans un milieu de culture, en présence du composé organique, exogène ou formé d'une manière intermédiaire, choisi parmi les cycloalcanes et cycloalcènes éventuellement substitués, et qui est un substrat de la mono-oxygénase ; ou
a2) on incube un milieu réactionnel contenant un substrat, avec une mono-oxygénase à cytochrome P450 selon l'une des revendications 1 à 3 ; et
b) on isole du milieu le produit d'oxydation formé, ou un dérivé de ce dernier.

12. Procédé selon la revendication 11, **caractérisé en ce qu'**on met en oeuvre l'oxydation par culture des micro-organismes en présence d'oxygène à une température de culture d'au moins environ 20°C et à un pH d'environ 6 à 9.

13. Procédé selon la revendication 11, **caractérisé en ce que**, en tant que substrat exogène, on ajoute à un milieu au moins un composé choisi parmi les cycloalcanes et cycloalcènes éventuellement substitués, et on met en oeuvre l'oxydation par réaction enzymatique du milieu contenant le substrat, en présence d'oxygène à une température d'au moins environ 20°C et à un pH d'environ 6 à 9, le milieu contenant le substrat contenant par ailleurs, par rapport au substrat, un excès molaire 10x à 100x d'équivalents de réduction.

14. Bioréacteur comprenant une enzyme selon l'une des revendications 1 à 3 ou un micro-organisme recombinant selon la revendication 8 sous forme immobilisée.

15. Utilisation d'une mono-oxygénase à cytochrome P450 selon l'une des revendications 1 à 3, d'un vecteur selon la revendication 7 ou d'un micro-organisme selon la revendication 8 pour l'oxydation microbiologique de cycloalcanes et de cycloalcènes éventuellement substitués.


### SEQUENZPROTOKOLL

<110> BASF Aktiengesellschaft
<120> Neue thermophile Cytochrom P450 Monooxygenasen
<130> M/41524
<590>
   <141>
<160> 10
<170> Patent In Ver. 2.1
<210> 1
   <211> 1170
   <212> DNA
   <213> Thermus thermophilus
<220>
   <221> CDS
   <222> (1)..(1170)
<400> 1
   atg aag cgc ctt tcc ctg agg gag gcc tgg ccc tac ctg aaa gac ctc    48
   Met Lys Arg Leu Ser Leu Arg Glu Ala Trp Pro Tyr Leu Lys Asp Leu
    1               5                  10                  15
   cag caa gat ccc ctc gcc gtc ctg ctg gcg tgg ggc cgg gcc cac ccc    96
   Gln Gln Asp Pro Leu Ala Val Leu Leu Ala Trp Gly Arg Ala His Pro
               20                  25                  30
   cgg ctc ttc ctt ccc ctg ccc cgc ttc ccc ctg gcc ctg atc ttt gac   144
   Arg Leu Phe Leu Pro Leu Pro Arg Phe Pro Leu Ala Leu Ile Phe Asp
           35                  40                  45
   ccc gag ggg gtg gag ggg gcg ctc ctc gcc gag ggg acc acc aag gcc   192
   Pro Glu Gly Val Glu Gly Ala Leu Leu Ala Glu Gly Thr Thr Lys Ala
       50                  55                  60
   acc ttc cag tac cgg gcc ctc tcc cgc ctc acg ggg agg ggc ctc ctc   240
   Thr Phe Gln Tyr Arg Ala Leu Ser Arg Leu Thr Gly Arg Gly Leu Leu
   65                  70                  75                  80
   acc gac tgg ggg gaa agc tgg aag gag gcg cgc aag gcc ctc aaa gac   288
   Thr Asp Trp Gly Glu Ser Trp Lys Glu Ala Arg Lys Ala Leu Lys Asp
                   85                  90                  95
   ccc ttc ctg ccg aag aac gtc cgc ggc tac cgg gag gcc atg gag gag   336
   Pro Phe Leu Pro Lys Asn Val Arg Gly Tyr Arg Glu Ala Met Glu Glu
               100                 105                 110
   gag gcc cgg gcc ttc ttc ggg gag tgg cgg ggg gag gag cgg gac ctg   384
   Glu Ala Arg Ala Phe Phe Gly Glu Trp Arg Gly Glu Glu Arg Asp Leu
           115                 120                 125
   gac cac gag atg ctc gcc ctc tcc ctg cgc ctc ctc ggg cgg gcc ctc   432
   Asp His Glu Met Leu Ala Leu Ser Leu Arg Leu Leu Gly Arg Ala Leu
       130                 135                 140
   ttc ggg aag ccc ctc tcc cca agc ctc gcg gag cac gcc ctt aag gcc   480
   Phe Gly Lys Pro Leu Ser Pro Ser Leu Ala Glu His Ala Leu Lys Ala
   145                 150                 155                 160
   ctg gac cgg atc atg gcc cag acc agg agc ccc ctg gcc ctc ctg gac    528
   Leu Asp Arg Ile Met Ala Gln Thr Arg Ser Pro Leu Ala Leu Leu Asp
                   165                 170                 175
   ctg gcc gcc gaa gcc cgc ttc cgg aag gac cgg ggg gcc ctc tac cgc    576
   Leu Ala Ala Glu Ala Arg Phe Arg Lys Asp Arg Gly Ala Leu Tyr Arg
               180                 185                 190
   gag gcg gaa gcc ctc atc gtc cac ccg ccc ctc tcc cac ctt ccc cga    624
   Glu Ala Glu Ala Leu Ile Val His Pro Pro Leu Ser His Leu Pro Arg
           195                 200                 205
   gag cgc gcc ctg agc gag gcc gtg acc ctc ctg gtg gcg ggc cac gag    672
   Glu Arg Ala Leu Ser Glu Ala Val Thr Leu Leu Val Ala Gly His Glu
       210                 215                 220
   acg gtg gcg agc gcc ctc acc tgg tcc ttt ctc ctc ctc tcc cac cgc    720
   Thr Val Ala Ser Ala Leu Thr Trp Ser Phe Leu Leu Leu Ser His Arg
   225                 230                 235                 240
   ccg gac tgg cag aag cgg gtg gcc gag agc gag gag gcg gcc ctc gcc    768
   Pro Asp Trp Gln Lys Arg Val Ala Glu Ser Glu Glu Ala Ala Leu Ala
                   245                 250                 255
   gcc ttc cag gag gcc ctg agg ctc tac ccc ccc gcc tgg atc ctc acc    816
   Ala Phe Gln Glu Ala Leu Arg Leu Tyr Pro Pro Ala Trp Ile Leu Thr
               260                 265                 270
   cgg agg ctg gaa agg ccc ctc ctc ctg gga gag gac cgg ctc ccc ccg    864
   Arg Arg Leu Glu Arg Pro Leu Leu Leu Gly Glu Asp Arg Leu Pro Pro
           275                 280                 285
   ggc acc acc ctg gtc ctc tcc ccc tac gtg acc cag agg ctc cac ttc    912
   Gly Thr Thr Leu Val Leu Ser Pro Tyr Val Thr Gln Arg Leu His Phe
       290                 295                 300
   ccc gat ggg gag gcc ttc cgg ccc gag cgc ttc ctg gag gaa agg ggg    960
   Pro Asp Gly Glu Ala Phe Arg Pro Glu Arg Phe Leu Glu Glu Arg Gly
   305                 310                 315                 320
   acc cct tcg ggg cgc tac ttc ccc ttt ggc ctg ggg cag agg ctc tgc    1008
   Thr Pro Ser Gly Arg Tyr Phe Pro Phe Gly Leu Gly Gln Arg Leu Cys
                   325                 330                 335
   ctg ggg cgg gac ttc gcc ctc ctc gag ggc ccc atc gtc ctc agg gcc    1056
   Leu Gly Arg Asp Phe Ala Leu Leu Glu Gly Pro Ile Val Leu Arg Ala
               340                 345                 350
   ttc ttc cgc cgc ttc cgc cta gac ccc ctc ccc ttc ccc cgg gtc ctc    1104
   Phe Phe Arg Arg Phe Arg Leu Asp Pro Leu Pro Phe Pro Arg Val Leu
           355                 360                 365
   gcc cag gtc acc ctg agg ccc gaa ggc ggg ctt ccc gcg cgg cct agg    1152
   Ala Gln Val Thr Leu Arg Pro Glu Gly Gly Leu Pro Ala Arg Pro Arg
       370                 375                 380
   gag gag gtg cgg gcg tga                                            1170
   Glu Glu Val Arg Ala
   385                 390
<210> 2
   <211> 389
   <212> PRT
   <213> Thermus thermophilus
<400> 2
   Met Lys Arg Leu Ser Leu Arg Glu Ala Trp Pro Tyr Leu Lys Asp Leu
   1               5                   10                  15
   Gln Gln Asp Pro Leu Ala Val Leu Leu Ala Trp Gly Arg Ala His Pro
               20                  25                  30
   Arg Leu Phe Leu Pro Leu Pro Arg Phe Pro Leu Ala Leu Ile Phe Asp
           35                  40                  45
   Pro Glu Gly Val Glu Gly Ala Leu Leu Ala Glu Gly Thr Thr Lys Ala
       50                  55                  60
   Thr Phe Gln Tyr Arg Ala Leu Ser Arg Leu Thr Gly Arg Gly Leu Leu
   65                  70                  75                  80
   Thr Asp Trp Gly Glu Ser Trp Lys Glu Ala Arg Lys Ala Leu Lys Asp
                   85                  90                  95
   Pro Phe Leu Pro Lys Asn Val Arg Gly Tyr Arg Glu Ala Met Glu Glu
               100                 105                 110
   Glu Ala Arg Ala Phe Phe Gly Glu Trp Arg Gly Glu Glu Arg Asp Leu
           115                 120                 125
   Asp His Glu Met Leu Ala Leu Ser Leu Arg Leu Leu Gly Arg Ala Leu
       130                 135                 140
   Phe Gly Lys Pro Leu Ser Pro Ser Leu Ala Glu His Ala Leu Lys Ala
   145                 150                 155                 160
   Leu Asp Arg Ile Met Ala Gln Thr Arg Ser Pro Leu Ala Leu Leu Asp
                   165                 170                 175
   Leu Ala Ala Glu Ala Arg Phe Arg Lys Asp Arg Gly Ala Leu Tyr Arg
               180                 185                 190
   Glu Ala Glu Ala Leu Ile Val His Pro Pro Leu Ser His Leu Pro Arg
           195                 200                 205
   Glu Arg Ala Leu Ser Glu Ala Val Thr Leu Leu Val Ala Gly His Glu
       210                 215                 220
   Thr Val Ala Ser Ala Leu Thr Trp Ser Phe Leu Leu Leu Ser His Arg
   225                 230                 235                 240
   Pro Asp Trp Gln Lys Arg Val Ala Glu Ser Glu Glu Ala Ala Leu Ala
                   245                 250                 255
   Ala Phe Gln Glu Ala Leu Arg Leu Tyr Pro Pro Ala Trp Ile Leu Thr
               260                 265                 270
   Arg Arg Leu Glu Arg Pro Leu Leu Leu Gly Glu Asp Arg Leu Pro Pro
           275                 280                 285
   Gly Thr Thr Leu Val Leu Ser Pro Tyr Val Thr Gln Arg Leu His Phe
       290                 295                 300
   Pro Asp Gly Glu Ala Phe Arg Pro Glu Arg Phe Leu Glu Glu Arg Gly
   305                 310                 315                 320
   Thr Pro Ser Gly Arg Tyr Phe Pro Phe Gly Leu Gly Gln Arg Leu Cys
                   325                 330                 335
   Leu Gly Arg Asp Phe Ala Leu Leu Glu Gly Pro Ile Val Leu Arg Ala
               340                 345                 350
   Phe Phe Arg Arg Phe Arg Leu Asp Pro Leu Pro Phe Pro Arg Val Leu
           355                 360                 365
   Ala Gln Val Thr Leu Arg Pro Glu Gly Gly Leu Pro Ala Arg Pro Arg
       370                 375                 380
   Glu Glu Val Arg Ala
   385
<210> 3
   <211> 1188
   <212> DNA
   <213> Künstliche Sequenz
<220>
   <221> misc feature
   <222> (4) .. (21)
   <223> His tag
<220>
   <223> Beschreibung der künstlichen Sequenz:N-terminal his tagged
<220>
   <221> CDS
   <222> (1)..(1188)
<400> 3
   atg cat cac cat cat cat cac aag cgc ctt tcc ctg agg gag gcc tgg    48
   Met His His His His His His Lys Arg Leu Ser Leu Arg Glu Ala Trp
    1               5                  10                  15
   ccc tac ctg aaa gac ctc cag caa gat ccc ctc gcc gtc ctg ctg gcg    96
   Pro Tyr Leu Lys Asp Leu Gln Gln Asp Pro Leu Ala Val Leu Leu Ala
               20                  25                  30
   tgg ggc cgg gcc cac ccc cgg ctc ttc ctt ccc ctg ccc cgc ttc ccc   144
   Trp Gly Arg Ala His Pro Arg Leu Phe Leu Pro Leu Pro Arg Phe Pro
           35                  40                  45
   ctg gcc ctg atc ttt gac ccc gag ggg gtg gag ggg gcg ctc ctc gcc   192
   Leu Ala Leu Ile Phe Asp Pro Glu Gly Val Glu Gly Ala Leu Leu Ala
       50                  55                  60
   gag ggg acc acc aag gcc acc ttc cag tac cgg gcc ctc tcc cgc ctc   240
   Glu Gly Thr Thr Lys Ala Thr Phe Gln Tyr Arg Ala Leu Ser Arg Leu
    65                  70                  75                  80
   acg ggg agg ggc ctc ctc acc gac tgg ggg gaa agc tgg aag gag gcg   288
   Thr Gly Arg Gly Leu Leu Thr Asp Trp Gly Glu Ser Trp Lys Glu Ala
                    85                  90                  95
   cgc aag gcc ctc aaa gac ccc ttc ctg ccg aag aac gtc cgc ggc tac     336
   Arg Lys Ala Leu Lys Asp Pro Phe Leu Pro Lys Asn Val Arg Gly Tyr
               100                 105                 110
   cgg gag gcc atg gag gag gag gcc cgg gcc ttc ttc ggg gag tgg cgg     384
   Arg Glu Ala Met Glu Glu Glu Ala Arg Ala Phe Phe Gly Glu Trp Arg
           115                 120                 125
   ggg gag gag cgg gac ctg gac cac gag atg ctc gcc ctc tcc ctg cgc     432
   Gly Glu Glu Arg Asp Leu Asp His Glu Met Leu Ala Leu Ser Leu Arg
       130                 135                 140
   ctc ctc ggg cgg gcc ctc ttc ggg aag ccc ctc tcc cca agc ctc gcg     480
   Leu Leu Gly Arg Ala Leu Phe Gly Lys Pro Leu Ser Pro Ser Leu Ala
   145                 150                 155                 160
   gag cac gcc ctt aag gcc ctg gac cgg atc atg gcc cag acc agg agc     528
   Glu His Ala Leu Lys Ala Leu Asp Arg Ile Met Ala Gln Thr Arg Ser
                   165                 170                 175
   ccc ctg gcc ctc ctg gac ctg gcc gcc gaa gcc cgc ttc cgg aag gac     576
   Pro Leu Ala Leu Leu Asp Leu Ala Ala Glu Ala Arg Phe Arg Lys Asp
               180                 185                 190
   cgg ggg gcc ctc tac cgc gag gcg gaa gcc ctc atc gtc cac ccg ccc     624
   Arg Gly Ala Leu Tyr Arg Glu Ala Glu Ala Leu Ile Val His Pro Pro
           195                 200                 205
   ctc tcc cac ctt ccc cga gag cgc gcc ctg agc gag gcc gtg acc ctc     672
   Leu Ser His Leu Pro Arg Glu Arg Ala Leu Ser Glu Ala Val Thr Leu
       210                 215                 220
   ctg gtg gcg ggc cac gag acg gtg gcg agc gcc ctc acc tgg tcc ttt     720
   Leu Val Ala Gly His Glu Thr Val Ala Ser Ala Leu Thr Trp Ser Phe
   225                 230                 235                 240
   ctc ctc ctc tcc cac cgc ccg gac tgg cag aag cgg gtg gcc gag agc     768
   Leu Leu Leu Ser His Arg Pro Asp Trp Gln Lys Arg Val Ala Glu Ser
                   245                 250                 255
   gag gag gcg gcc ctc gcc gcc ttc cag gag gcc ctg agg ctc tac ccc     816
   Glu Glu Ala Ala Leu Ala Ala Phe Gln Glu Ala Leu Arg Leu Tyr Pro
               260                 265                 270
   ccc gcc tgg atc ctc acc cgg agg ctg gaa agg ccc ctc ctc ctg gga     864
   Pro Ala Trp Ile Leu Thr Arg Arg Leu Glu Arg Pro Leu Leu Leu Gly
           275                 280                 285
   gag gac cgg ctc ccc ccg ggc acc acc ctg gtc ctc tcc ccc tac gtg     912
   Glu Asp Arg Leu Pro Pro Gly Thr Thr Leu Val Leu Ser Pro Tyr Val
       290                 295                 300
   acc cag agg ctc cac ttc ccc gat ggg gag gcc ttc cgg ccc gag cgc     960
   Thr Gln Arg Leu His Phe Pro Asp Gly Glu Ala Phe Arg Pro Glu Arg
   305                 310                 315                 320
   ttc ctg gag gaa agg ggg acc cct tcg ggg cgc tac ttc ccc ttt ggc     1008
   Phe Leu Glu Glu Arg Gly Thr Pro Ser Gly Arg Tyr Phe Pro Phe Gly
                   325                 330                 335
   ctg ggg cag agg ctc tgc ctg ggg cgg gac ttc gcc ctc ctc gag ggc     1056
   Leu Gly Gln Arg Leu Cys Leu Gly Arg Asp Phe Ala Leu Leu Glu Gly
               340                 345                 350
   ccc atc gtc ctc agg gcc ttc ttc cgc cgc ttc cgc cta gac ccc ctc     1104
   Pro Ile Val Leu Arg Ala Phe Phe Arg Arg Phe Arg Leu Asp Pro Leu
           355                 360                 365
   ccc ttc ccc cgg gtc ctc gcc cag gtc acc ctg agg ccc gaa ggc ggg     1152
   Pro Phe Pro Arg Val Leu Ala Gln Val Thr Leu Arg Pro Glu Gly Gly
       370                 375                 380
   ctt ccc gcg cgg cct agg gag gag gtg cgg gcg tga                     1188
   Leu Pro Ala Arg Pro Arg Glu Glu Val Arg Ala
   385                 390                 395
<210> 4
   <211> 395
   <212> PRT
   <213> Künstliche Sequenz
   <223> Beschreibung der künstlichen Sequenz:N-terminal his tagged
<400> 4
   Met His His His His His His Lys Arg Leu Ser Leu Arg Glu Ala Trp
    1               5                  10                  15
   Pro Tyr Leu Lys Asp Leu Gln Gln Asp Pro Leu Ala Val Leu Leu Ala
               20                  25                  30
   Trp Gly Arg Ala His Pro Arg Leu Phe Leu Pro Leu Pro Arg Phe Pro
           35                  40                  45
   Leu Ala Leu Ile Phe Asp Pro Glu Gly Val Glu Gly Ala Leu Leu Ala
       50                  55                  60
   Glu Gly Thr Thr Lys Ala Thr Phe Gln Tyr Arg Ala Leu Ser Arg Leu
   65                  70                  75                      80
   Thr Gly Arg Gly Leu Leu Thr Asp Trp Gly Glu Ser Trp Lys Glu Ala
                   85                  90                  95
   Arg Lys Ala Leu Lys Asp Pro Phe Leu Pro Lys Asn Val Arg Gly Tyr
               100                 105                 110
   Arg Glu Ala Met Glu Glu Glu Ala Arg Ala Phe Phe Gly Glu Trp Arg
           115                 120                 125
   Gly Glu Glu Arg Asp Leu Asp His Glu Met Leu Ala Leu Ser Leu Arg
       130                 135                 140
   Leu Leu Gly Arg Ala Leu Phe Gly Lys Pro Leu Ser Pro Ser Leu Ala
   145                 150                 155                     160
   Glu His Ala Leu Lys Ala Leu Asp Arg Ile Met Ala Gln Thr Arg Ser
                   165                 170                 175
   Pro Leu Ala Leu Leu Asp Leu Ala Ala Glu Ala Arg Phe Arg Lys Asp
               180                 185                 190
   Arg Gly Ala Leu Tyr Arg Glu Ala Glu Ala Leu Ile Val His Pro Pro
           195                 200                 205
   Leu Ser His Leu Pro Arg Glu Arg Ala Leu Ser Glu Ala Val Thr Leu
       210                 215                 220
   Leu Val Ala Gly His Glu Thr Val Ala Ser Ala Leu Thr Trp Ser Phe
   225                 230                 235                 240
   Leu Leu Leu Ser His Arg Pro Asp Trp Gln Lys Arg Val Ala Glu Ser
                   245                 250                 255
   Glu Glu Ala Ala Leu Ala Ala Phe Gln Glu Ala Leu Arg Leu Tyr Pro
               260                 265                 270
   Pro Ala Trp Ile Leu Thr Arg Arg Leu Glu Arg Pro Leu Leu Leu Gly
           275                 280                 285
   Glu Asp Arg Leu Pro Pro Gly Thr Thr Leu Val Leu Ser Pro Tyr Val
       290                 295                 300
   Thr Gln Arg Leu His Phe Pro Asp Gly Glu Ala Phe Arg Pro Glu Arg
   305                 310                 315                 320
   Phe Leu Glu Glu Arg Gly Thr Pro Ser Gly Arg Tyr Phe Pro Phe Gly
                   325                 330                 335
   Leu Gly Gln Arg Leu Cys Leu Gly Arg Asp Phe Ala Leu Leu Glu Gly
               340                 345                 350
   Pro Ile Val Leu Arg Ala Phe Phe Arg Arg Phe Arg Leu Asp Pro Leu
           355                 360                 365
   Pro Phe Pro Arg Val Leu Ala Gln Val Thr Leu Arg Pro Glu Gly Gly
       370                 375                 380
   Leu Pro Ala Arg Pro Arg Glu Glu Val Arg Ala
   385                 390                 395
<210> 5
   <211> 1188
   <212> DNA
   <213> Künstliche Sequenz
<220>
   <221> misc_feature
   <222> (1168)..(1185)
   <223> His tag
<220>
   <223> Beschreibung der künstlichen Sequenz:C-terminal His-tagged
<220>
   <221> CDS
   <222> (1)..(1188)
<400> 5
   atg aag cgc ctt tcc ctg agg gag gcc tgg ccc tac ctg aaa gac ctc     48
   Met Lys Arg Leu Ser Leu Arg Glu Ala Trp Pro Tyr Leu Lys Asp Leu
     1               5                  10                  15
   cag caa gat ccc ctc gcc gtc ctg ctg gcg tgg ggc cgg gcc cac ccc     96
   Gln Gln Asp Pro Leu Ala Val Leu Leu Ala Trp Gly Arg Ala His Pro
                20                  25                  30
   cgg ctc ttc ctt ccc ctg ccc cgc ttc ccc ctg gcc ctg atc ttt gac     144
   Arg Leu Phe Leu Pro Leu Pro Arg Phe Pro Leu Ala Leu Ile Phe Asp
           35                  40                  45
   ccc gag ggg gtg gag ggg gcg ctc ctc gcc gag ggg acc acc aag gcc     192
   Pro Glu Gly Val Glu Gly Ala Leu Leu Ala Glu Gly Thr Thr Lys Ala
       50                  55                  60
   acc ttc cag tac cgg gcc ctc tcc cgc ctc acg ggg agg ggc ctc ctc     240
   Thr Phe Gln Tyr Arg Ala Leu Ser Arg Leu Thr Gly Arg Gly Leu Leu
   65                  70                  75                  80
   acc gac tgg ggg gaa agc tgg aag gag gcg cgc aag gcc ctc aaa gac     288
   Thr Asp Trp Gly Glu Ser Trp Lys Glu Ala Arg Lys Ala Leu Lys Asp
                   85                  90                  95
   ccc ttc ctg ccg aag aac gtc cgc ggc tac cgg gag gcc atg gag gag     336
   Pro Phe Leu Pro Lys Asn Val Arg Gly Tyr Arg Glu Ala Met Glu Glu
               100                 105                 110
   gag gcc cgg gcc ttc ttc ggg gag tgg cgg ggg gag gag cgg gac ctg     384
   Glu Ala Arg Ala Phe Phe Gly Glu Trp Arg Gly Glu Glu Arg Asp Leu
           115                 120                 125
   gac cac gag atg ctc gcc ctc tcc ctg cgc ctc ctc ggg cgg gcc ctc     432
   Asp His Glu Met Leu Ala Leu Ser Leu Arg Leu Leu Gly Arg Ala Leu
       130                 135                 140
   ttc ggg aag ccc ctc tcc cca agc ctc gcg gag cac gcc ctt aag gcc     480
   Phe Gly Lys Pro Leu Ser Pro Ser Leu Ala Glu His Ala Leu Lys Ala
   145                 150                 155                 160
   ctg gac cgg atc atg gcc cag acc agg agc ccc ctg gcc ctc ctg gac     528
   Leu Asp Arg Ile Met Ala Gln Thr Arg Ser Pro Leu Ala Leu Leu Asp
                   165                 170                 175
   ctg gcc gcc gaa gcc cgc ttc cgg aag gac cgg ggg gcc ctc tac cgc     576
   Leu Ala Ala Glu Ala Arg Phe Arg Lys Asp Arg Gly Ala Leu Tyr Arg
               180                 185                 190
   gag gcg gaa gcc ctc atc gtc cac ccg ccc ctc tcc cac ctt ccc cga     624
   Glu Ala Glu Ala Leu Ile Val His Pro Pro Leu Ser His Leu Pro Arg
           195                 200                 205
   gag cgc gcc ctg agc gag gcc gtg acc ctc ctg gtg gcg ggc cac gag     672
   Glu Arg Ala Leu Ser Glu Ala Val Thr Leu Leu Val Ala Gly His Glu
       210                 215                 220
   acg gtg gcg agc gcc ctc acc tgg tcc ttt ctc ctc ctc tcc cac cgc     720
   Thr Val Ala Ser Ala Leu Thr Trp Ser Phe Leu Leu Leu Ser His Arg
   225                 230                 235                 240
   ccg gac tgg cag aag cgg gtg gcc gag agc gag gag gcg gcc ctc gcc     768
   Pro Asp Trp Gln Lys Arg Val Ala Glu Ser Glu Glu Ala Ala Leu Ala
                   245                 250                 255
   gcc ttc cag gag gcc ctg agg ctc tac ccc ccc gcc tgg atc ctc acc     816
   Ala Phe Gln Glu Ala Leu Arg Leu Tyr Pro Pro Ala Trp Ile Leu Thr
               260                 265                 270
   cgg agg ctg gaa agg ccc ctc ctc ctg gga gag gac cgg ctc ccc ccg     864
   Arg Arg Leu Glu Arg Pro Leu Leu Leu Gly Glu Asp Arg Leu Pro Pro
           275                 280                 285
   ggc acc acc ctg gtc ctc tcc ccc tac gtg acc cag agg ctc cac ttc      912
   Gly Thr Thr Leu Val Leu Ser Pro Tyr Val Thr Gln Arg Leu His Phe
       290                 295                 300
   ccc gat ggg gag gcc ttc cgg ccc gag cgc ttc ctg gag gaa agg ggg      960
   Pro Asp Gly Glu Ala Phe Arg Pro Glu Arg Phe Leu Glu Glu Arg Gly
   305                 310                 315                 320
   acc cct tcg ggg cgc tac ttc ccc ttt ggc ctg ggg cag agg ctc tgc      1008
   Thr Pro Ser Gly Arg Tyr Phe Pro Phe Gly Leu Gly Gln Arg Leu Cys
                   325                 330                 335
   ctg ggg cgg gac ttc gcc ctc ctc gag ggc ccc atc gtc ctc agg gcc      1056
   Leu Gly Arg Asp Phe Ala Leu Leu Glu Gly Pro Ile Val Leu Arg Ala
               340                 345                 350
   ttc ttc cgc cgc ttc cgc cta gac ccc ctc ccc ttc ccc cgg gtc ctc      1104
   Phe Phe Arg Arg Phe Arg Leu Asp Pro Leu Pro Phe Pro Arg Val Leu
           355                 360                 365
   gcc cag gtc acc ctg agg ccc gaa ggc ggg ctt ccc gcg cgg cct agg      1152
   Ala Gln Val Thr Leu Arg Pro Glu Gly Gly Leu Pro Ala Arg Pro Arg
       370                 375                 380
   gag gag gtg cgg gcg cat cac cat cat cat cac tga                      1188
   Glu Glu Val Arg Ala His His His His His His
   385                 390                 395
<210> 6
   <211> 395
   <212> PRT
   <213> Künstliche Sequenz
   <223> Beschreibung der künstlichen Sequenz:C-terminal His-tagged
<400> 6
   Met Lys Arg Leu Ser Leu Arg Glu Ala Trp Pro Tyr Leu Lys Asp Leu
    1               5                  10                  15
   Gln Gln Asp Pro Leu Ala Val Leu Leu Ala Trp Gly Arg Ala His Pro
               20                  25                  30
   Arg Leu Phe Leu Pro Leu Pro Arg Phe Pro Leu Ala Leu Ile Phe Asp
           35                  40                  45
   Pro Glu Gly Val Glu Gly Ala Leu Leu Ala Glu Gly Thr Thr Lys Ala
       50                  55                  60
   Thr Phe Gln Tyr Arg Ala Leu Ser Arg Leu Thr Gly Arg Gly Leu Leu
   65                  70                  75                  80
   Thr Asp Trp Gly Glu Ser Trp Lys Glu Ala Arg Lys Ala Leu Lys Asp
                   85                  90                  95
   Pro Phe Leu Pro Lys Asn Val Arg Gly Tyr Arg Glu Ala Met Glu Glu
               100                 105                 110
   Glu Ala Arg Ala Phe Phe Gly Glu Trp Arg Gly Glu Glu Arg Asp Leu
           115                 120                 125
   Asp His Glu Met Leu Ala Leu Ser Leu Arg Leu Leu Gly Arg Ala Leu
       130                 135                 140
   Phe Gly Lys Pro Leu Ser Pro Ser Leu Ala Glu His Ala Leu Lys Ala
   145                 150                 155                 160
   Leu Asp Arg Ile Met Ala Gln Thr Arg Ser Pro Leu Ala Leu Leu Asp
                   165                 170                 175
   Leu Ala Ala Glu Ala Arg Phe Arg Lys Asp Arg Gly Ala Leu Tyr Arg
               180                 185                 190
   Glu Ala Glu Ala Leu Ile Val His Pro Pro Leu Ser His Leu Pro Arg
           195                 200                 205
   Glu Arg Ala Leu Ser Glu Ala Val Thr Leu Leu Val Ala Gly His Glu
       210                 215                 220
   Thr Val Ala Ser Ala Leu Thr Trp Ser Phe Leu Leu Leu Ser His Arg
   225                 230                 235                 240
   Pro Asp Trp Gln Lys Arg Val Ala Glu Ser Glu Glu Ala Ala Leu Ala
                   245                 250                 255
   Ala Phe Gln Glu Ala Leu Arg Leu Tyr Pro Pro Ala Trp Ile Leu Thr
               260                 265                 270
   Arg Arg Leu Glu Arg Pro Leu Leu Leu Gly Glu Asp Arg Leu Pro Pro
           275                 280                 285
   Gly Thr Thr Leu Val Leu Ser Pro Tyr Val Thr Gln Arg Leu His Phe
       290                 295                 300
   Pro Asp Gly Glu Ala Phe Arg Pro Glu Arg Phe Leu Glu Glu Arg Gly
   305                 310                 315                 320
   Thr Pro Ser Gly Arg Tyr Phe Pro Phe Gly Leu Gly Gln Arg Leu Cys
                   325                 330                 335
   Leu Gly Arg Asp Phe Ala Leu Leu Glu Gly Pro Ile Val Leu Arg Ala
               340                 345                 350
   Phe Phe Arg Arg Phe Arg Leu Asp Pro Leu Pro Phe Pro Arg Val Leu
           355                 360                 365
   Ala Gln Val Thr Leu Arg Pro Glu Gly Gly Leu Pro Ala Arg Pro Arg
       370                 375                 380
   Glu Glu Val Arg Ala His His His His His His
   385                 390                 395
<210> 7
   <211> 30
   <212> DNA
   <213> Künstliche Sequenz
<220>
   <223> Beschreibung der künstlichen Sequenz:PC:R-Primer
<400> 7
   cgaagctcat atgaagcgcc tttccctgag 30
<210> 8
   <211> 30
   <212> DNA
   <213> Künstliche Sequenz
<220>
   <223> Beschreibung der künstlichen Sequenz:PCR-Primer
<400> 8
   gcgaattcac gcccgcacct cctccctagg 30
<210> 9
   <211> 42
   <212> DNA
   <213> Künstliche Sequenz
<220>
   <223> Beschreibung der künstlichen Sequenz:PCR-Primer
<900> 9
   cgaagctcat atgcatcacc atcatcatca caagcgcctt tc 42
<210> 10
   <211> 42
   <212> DNA
   <213> Künstliche Sequenz
<220>
   <223> Beschreibung der künstlichen Sequenz:PCR-Primer
<400> 10
   cggaattcag tgatgatgat ggtgatgcgc ccgcacctcc tc 42